# EUROPEAN PATENT SPECIFICATION

(11) **EP 3 106 091 B1**
(45) Date of publication and mention of the grant of the patent: **03.10.2018**
(21) Application number: 16173909.9
(22) Date of filing: 10.06.2016
(51) Int. Cl.: A61B 5/107, A61B 5/00, A61B 5/103

(54) **APPARATUS FOR ASSESSING THE FUNCTIONAL DYSMETRIA OF THE LOWER LIMBS**
VORRICHTUNG ZUR BEURTEILUNG DER FUNKTIONALEN DYSMETRIE DER UNTEREN GLIEDMASSEN
APPAREIL POUR EVALUER LA DYSMETRIE FONCTIONNELLE DES MEMBRES INFERIEURS

(30) Priority: 15.06.2015 IT UB20151386
(43) Date of publication of application: 21.12.2016
(73) Proprietor: Sponsor S.r.l., 31044 Montebelluna, Frazione Biadene (TV) (IT)
(72) Inventor: GRASSI, Silvano, 31044 Montebelluna TV (IT)
(74) Representative: Modiano, Micaela Nadia

(56) References cited:
- EP-A2- 0 666 038
- US-A- 5 027 461
- US-A- 5 823 974
- US-B1- 6 962 568

## Description

The present invention relates to an apparatus for assessing the functional dysmetria of the lower limbs.

Nowadays devices are known for assessing the dysmetria of the lower limbs, which substantially comprise two moveable platforms that are mutually independent, mounted on a conventional base and can yield vertically under load.

In particular, apparatus of this type are disclosed in European patent application EP0666038. According to the description thereof, the pliability of the platforms is obtained by placing each one of them on an elastically deformable medium, such as a block shaped substantially like a parallelepiped which is made of expanded plastic material and flexible.

Each platform is subdivided transversely into two sectors, one for resting the heel, the other for resting the forefoot, which can rotate independently of each other, accompanying any anomalies in the arrangement of the foot about a longitudinal central axis of the platform.

The platforms cooperate with means for the vertical arrangement, with respect to the foot-resting central line, of the sacrolumbar line of the patient and with means for measuring the dysmetria.

Such means for arrangement consist of sliders, which are moveable on an adapted structure, for the coccygeal and cervical centering along a common vertical line, and such means for measurement consist of a graduated scale that conveniently complements the apparatus.

This apparatus makes it possible to position the patient affected by dysmetria on the platforms, making the patient assume a position with the coccyx symmetrically equidistant with respect to the placing of the feet.

In these conditions the patient affected by dysmetria presents a load on the two limbs which is different and which can be seen in the fact that one platform yields differently to the other, so that the resting surfaces of the feet are at different levels.

The difference in level constitutes the dysmetria, which can be conveniently measured.

Although it has been found in technical terms to be valid and it is simple and inexpensive to make, such apparatus is not devoid of drawbacks.

The most prominent is constituted by the fact that the pliability of the platforms is obtained by way of blocks made of flexible expanded material, which have to be substituted under the platforms with other of different density, so as to adjust the system to the load conditions, which are different from patient to patient, in terms of the weight of the patient.

The choice of new blocks and their insertion under the platforms constituted by the two mentioned sections is laborious owing to the omnidirectional mobility thereof.

Furthermore, the possible rotation of the two sectors of the platform about the longitudinal central axis renders such platform unstable, and therefore makes it difficult for the patient to find a comfortable balance, which is essential in order to proceed with the measuring of the dysmetria, and even more so if the patient is wearing shoes.

The use of shoes, even with high heels, would make it possible to reproduce real conditions with respect to the ground, which, however, does not tilt like the two sectors of each platform.

Some of these drawbacks have been overcome by way of an apparatus the structure of which is disclosed in United States patent US5823974.

In this apparatus each block of flexible expanded material is substituted by at least one respective pneumatic actuator, obtaining the necessary pliability by way of dosing the internal pressure, and there are also pneumatic pistons for actuating the block of the platforms in the phase when the patient steps on, and potentiometers for reading the dysmetria.

The platforms have a vertical excursion that is strictly parallel to the ground, in this manner simulating the floor.

This solution also is conveniently provided with means for the vertical arrangement of the coccyx.

This solution makes it possible to obtain an optimal measurement of the dysmetria, and it is practical in use.

However, the use of a pneumatic portion is not convenient, owing to the need to provide the apparatus with a compressor, pneumatic dampers actuated by an adapted sophisticated electronic apparatus with corresponding software, cannulas for the air, etc., all of which make it expensive, structurally complex and, as a consequence, subject to maintenance and repair.

The aim of the present invention is to provide an apparatus for assessing the functional dysmetria of the lower limbs which eliminates the above mentioned drawbacks in conventional devices.

Within this aim, an object of the invention is to provide an apparatus that is structurally simple and easy to use, both for operators, who are assigned to use it, and for patients.

The invention relates to an apparatus for assessing the functional dysmetria of the lower limbs, characterized in that it comprises:
- a supporting structure with two moveable platforms, which are mutually independent and can yield vertically under load, at least one block made of elastically yielding material being arranged below each one of said platforms,
- complementary means, which cooperate with said platforms, for the vertical arrangement, with respect to the foot-resting central line, of the sacrolumbar line of the patient,
- means for measuring dysmetria,
- means for measuring the differences in subcalcaneal load,
- means for locking and releasing said platforms,
- means for extracting the blocks from said supporting structure,
wherein said extraction means comprise a slider which supports said blocks and can be extracted from said supporting structure by sliding in a direction that is substantially parallel to the ground.

Further characteristics and advantages of the invention will become better apparent from the description of a preferred, but not exclusive, embodiment of the apparatus according to the invention, which is illustrated by way of non-limiting example in the accompanying drawings wherein:
- Figure 1 is a perspective view of the complete apparatus according to the invention;
- Figure 2 is a perspective view of the apparatus according to the invention in which the blocks can be seen;
- Figure 3 shows the apparatus according to the invention during extraction of the blocks;
- Figure 4 shows some internal parts of the apparatus according to the invention, specifically the means for locking and releasing the platforms.

With reference to the figures, the apparatus according to the invention is generally designated with the reference numeral 10 and comprises a supporting structure 11 with a base 12, for resting on the ground, which is a substantially rectangular parallelepiped.

The supporting structure 11 carries two moveable platforms 13, which are mutually independent and can yield vertically under load.

Positioned below each one of the platforms 13 is a block 14 made of elastically yielding material.

The block 14 is substantially parallelepiped in shape, preferably made of a flexible expanded plastic material.

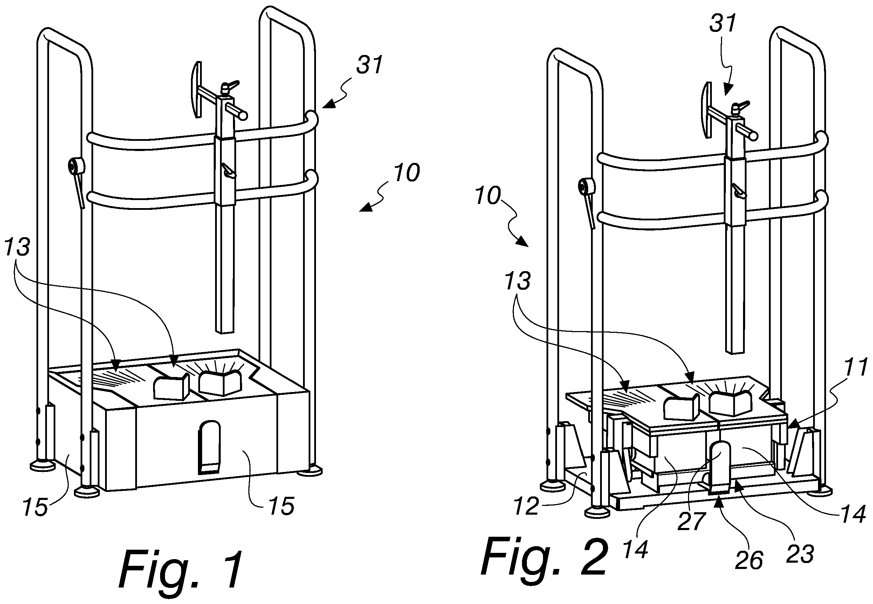

In Figure 1 the supporting structure 11 is not visible because it is covered by outer panels 15, while in the subsequent figures the apparatus 10 is shown without such panels 15, in order to show the internal part thereof, where the blocks 14 are positioned.

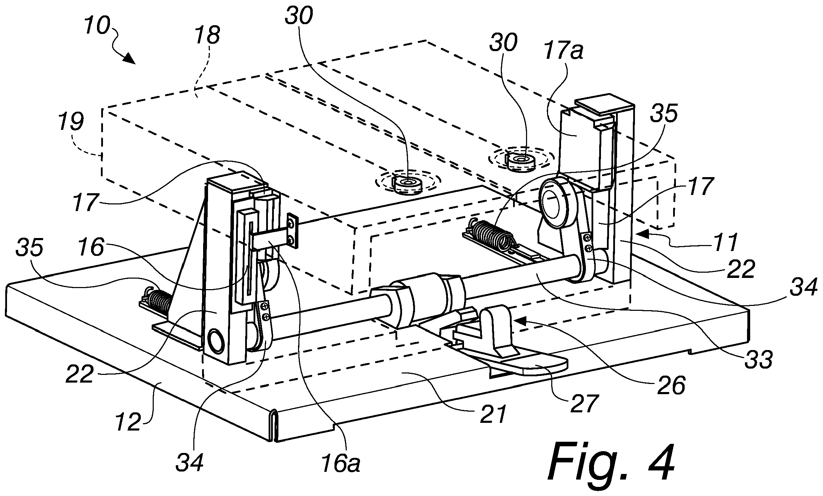

The apparatus 10 comprises means 16 for measuring the dysmetria, and vertical sliding guides 17 for the platforms 13, which are shown in Figure 4.

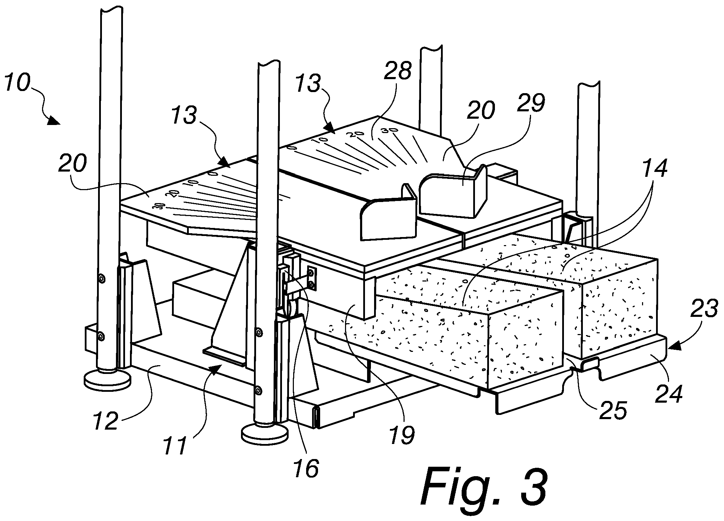

As can be seen in Figure 3 and in Figure 4, the platforms 13 each comprise a horizontal surface 18 with a side wall 19, on the outer part, and a resting surface 20, which is superimposed on the horizontal surface 18, for the foot of the patient. The two blocks 14 are positioned substantially between the two side walls 19 of the two platforms 13.

In the example shown, the measurement means 16 and the guides 17 are installed at each side wall 19. They are indicated in Figure 4, in which the surfaces 18 and the side walls 19 of the platforms 13 are shown transparent so as not to hide such elements.

The supporting structure 11 comprises a crossmember 21 fixed to the resting base 12 and two uprights 22 extending vertically at the ends of the crossmember 21 at opposite sides of the supporting structure 11.

A linear guide 17 is installed on each upright 22. The two guides 17, at the two opposite sides of the supporting structure 11, have mutually parallel longitudinal vertical axes.

Each platform 13 slides on the respective guide 17 by way of a recirculating ball slider 17a which is rendered integral with the side wall 19 of the platform 13, on the outer part.

The measurement means 16 comprise, in the embodiment shown, for each platform 13, a linear potentiometer for reading the dysmetria.

Each potentiometer is installed on the respective upright 22 and is conveniently connected to conventional transduction devices and a software program for processing the data, which is capable of informing the operator of the difference between the two levels detected by way of the two opposing potentiometers, which constitutes the dysmetria of the patient.

The measurement means 16 each conveniently also comprise a slider element 16a which is rendered integral with the side wall 19, and the potentiometer is adapted to detect its movement.

The apparatus 10 according to the invention also comprises means 23 for extracting the blocks 14 from the supporting structure 11. Such extraction means 23 substantially consist of a slider 24 which supports the blocks 14 on its bottom 25 and which can be extracted from the supporting structure 11 by sliding in a direction that is substantially parallel to the ground.

The apparatus 10 conveniently also comprises means 26 for locking and releasing the platforms 13.

The locking and release means 26 comprise a lever, or pedal, 27 which in the vertical position prevents the platforms 13 from yielding, by acting on a shaft 33 which is pivoted to the two opposing uprights 22, two opposing linkages 34 being coupled to the shaft 33 which are adapted to keep the vertical sliding of the platforms 13 locked, the lower edge of a corresponding side wall 19 of the platforms 13 resting on the upper end of each linkage 34.

Acting on the lever or pedal causes the rotation of the shaft 33 and with it the linkages 34 which, by rotating, allow the platforms 13 to slide vertically.

The return to the initial position of the platforms 13 is done by the elastic thrust of the blocks 14 in cooperation with return springs 35, which by way of a belt 36 connected to a corresponding linkage 34 make the respective linkage 34 rotate in the direction to restore the initial position.

The lever or pedal 27 is kept in the vertical position by way of magnets which are integral therewith and which interact with a metallic portion of the resting base 12.

Figures 1 and 2 show the apparatus 10 with the slider 24 not extracted from the supporting structure 11 and with the lever 27 in the vertical position. Figure 3 shows the apparatus 10 with the slider 24 extracted, in which position it is possible to easily substitute the blocks 14.

Figure 3 and Figure 4 clearly show the resting surface 20 and the underlying horizontal surface 18 for each platform 13.

The platforms 13, in particular at the respective resting surface 20, are provided, on the upper surface for resting the foot, with a graduated scale 28 for measuring the degree of divarication of the feet and also with heel units 29 for positioning the feet correctly.

The horizontal surface 18 of each platform 13 is provided with a load cell 30, of conventional type, in the region that corresponds to the resting of the heel, which is adapted to detect the weight of the patient who steps onto the respective platform 13.

The apparatus 10 also conveniently comprises complementary means 31, which cooperate with the platforms 13, for the vertical arrangement, with respect to the foot-resting central line, of the sacrolumbar line of the patient.

Such complementary means 31, shown in the first two figures, are conventional and comprise means of vertical positioning, which consist of moveable sliders on an adapted vertical structure, for coccygeal centering.

Use of the apparatus, according to the invention, is the following.

The patient is made to step onto the locked platforms 13, and is made to position their feet with the same front angle and with the heel at the heel unit 29, thus showing the difference in subcalcaneal load.

For a correct measurement, with the platforms released, it is necessary to place the sacrolumbar line of the patient exactly vertical with respect to the foot-resting central line, by acting on the complementary means 31.

The operator who performs the measurement can draw on blocks 14 of different density and of known load capacity, from which to choose the one that is most suitable as a function of the load conditions, which vary from patient to patient.

The slider 24 is then extracted and the blocks can be easily substituted with blocks that are more suitable for the patient.

In these conditions, for a patient affected by dysmetria, the load on the two limbs is different and therefore one platform yields differently to the other, so that the resting surfaces 20 of the feet are at different levels. The blocks 14 are compressed by the weight of the patient and their pliability determines a descent of the platforms 13 which is instantaneously read by the measurement means 16: by way of the potentiometers and the corresponding transducer devices, the operator can read on the screen of a hand-held device or of a computer the difference between the two levels, calculated by an adapted supporting computer program, which constitutes the dysmetria of the patient.

The decreased difference of the subcalcaneal weight reported by the adapted load cells 30 confirms the correctness of any detected value of the dysmetria.

The apparatus according to the invention makes it possible to perform a measurement of dysmetria rapidly and exactly without using pneumatic components.

The apparatus is therefore relatively inexpensive to make and maintain and it is also structurally simple.

It makes it possible to use blocks made of expanded material, by substituting them with ease as a function of the characteristics of the patient.

Furthermore, the structure has stable resting surfaces for the patient, not tilting ones, and therefore it can be used even if wearing shoes with high heels, thus reproducing real conditions with respect to the ground.

In practice it has been found that the invention fully achieves the set aim and object by providing an apparatus that is inexpensive and easy to use, both for operators, who are assigned to use it, and for patients, who arrive at the most satisfactory equilibrium and relaxation on their own.

The invention, thus conceived, is susceptible of numerous modifications and variations, all of which are within the scope of the appended claims. Moreover, all the details may be substituted by other, technically equivalent elements.

In practice the materials employed, provided they are compatible with the specific use, and the contingent dimensions and shapes, may be any according to requirements and to the state of the art.

## Claims

1. An apparatus (10) for assessing the functional dysmetria of lower limbs, the apparatus (10) comprising:
- a supporting structure (11) with two moveable platforms (13), which are mutually independent and can yield vertically under load, at least one block (14) made of elastically yielding material being arranged below each one of said platforms,
- complementary means (31), which cooperate with said platforms (13), for the vertical arrangement, with respect to the foot-resting central line, of the sacrolumbar line of the patient,
- means (16) for measuring dysmetria,
- means (30) for measuring the differences in subcalcaneal load,
- means (26) for locking and releasing said platforms (13),
- means (23) for extracting the blocks (14) from said supporting structure (11),
**characterized in that** said extraction means (23) comprise a slider (24) which supports said blocks (14) and can be extracted from said supporting structure (11) by sliding in a direction that is substantially parallel to the ground.

2. The apparatus according to claim 1, **characterized in that** each one of the platforms (13) comprises a horizontal surface (18) with a side wall (19), on the outer part, and a resting surface (20), which is superimposed on the horizontal surface (18), for the foot of the patient, which lie above two blocks (14) which are arranged substantially between the two side walls (19) of the two platforms (13).

3. The apparatus according to one or more of the preceding claims, **characterized in that** said horizontal surface (18) of each platform (13) is provided with a load cell (30), in the region that corresponds to the resting of the heel, which is adapted to detect the weight of the patient who steps onto the respective platform (13).

4. The apparatus according to one or more of the preceding claims, **characterized in that** said measurement means (16) comprise, for each one of said platforms (13), a potentiometer for reading dysmetria.

5. The apparatus according to one or more of the preceding claims, **characterized in that** it comprises guides (17) for the vertical sliding of said platforms (13).

6. The apparatus according to one or more of the preceding claims, **characterized in that** said platforms (13) have, on the upper foot-resting surface, a graduated scale (28) for indicating the degree of divarication of the feet and the heel units (29) for their exact positioning.

## Patentansprüche

1. Eine Vorrichtung (10) zur Beurteilung der funktionalen Dysmetrie unterer Gliedmaßen, wobei die Vorrichtung (10) Folgendes umfasst:
- eine Stützstruktur (11) mit zwei beweglichen Plattformen (13), die voneinander unabhängig sind und vertikal unter Last nachgeben können, wobei mindestens ein Block (14) aus elastisch nachgebendem Material unterhalb jeder der Plattformen angeordnet ist,
- komplementäre Mittel (31), die mit den Plattformen (13) zur vertikalen Anordnung der sakrolumbalen Linie des Patienten im Verhältnis zur zentralen Fußauflagelinie zusammenwirken,
- Mittel (16) zur Messung der Dysmetrie,
- Mittel (30) zur Messung der Unterschiede in der subkalkanealen Last,
- Mittel (26) zum Blockieren und Lösen der Plattformen (13),
- Mittel (23) zum Herausziehen der Blöcke (14) aus der Stützstruktur (11),
**dadurch gekennzeichnet, dass** die Extraktionsmittel (23) einen Schieber (24) umfassen, der die Blöcke (14) trägt und durch Gleiten in eine Richtung, die im Wesentlichen parallel zum Boden ist, aus der Stützstruktur (11) herausgezogen werden kann.

2. Die Vorrichtung gemäß Anspruch 1, **dadurch gekennzeichnet, dass** jede der Plattformen (13) eine horizontale Oberfläche (18) mit einer Seitenwand (19) am äußeren Teil und einer Auflagefläche (20) umfasst, die für den Fuß des Patienten auf die horizontale Oberfläche (18) gelegt ist; die über zwei Blöcken (14) liegen, welche im Wesentlichen zwischen den beiden Seitenwänden (19) der beiden Plattformen (13) angeordnet sind.

3. Die Vorrichtung gemäß einem oder mehreren der obigen Ansprüche, **dadurch gekennzeichnet, dass** die horizontale Oberfläche (18) jeder Plattform (13) in dem Bereich, der dem Aufliegen der Ferse entspricht, mit einer Kraftmessdose (30) ausgestattet ist, die ausgebildet ist, um das Gewicht des Patienten zu messen, der auf die entsprechende Plattform (13) steigt.

4. Die Vorrichtung gemäß einem oder mehreren der obigen Ansprüche, **dadurch gekennzeichnet, dass** die Messmittel (16) für jede der Plattformen (13) ein Potentiometer zum Ablesen der Dysmetrie umfassen.

5. Die Vorrichtung gemäß einem oder mehreren der obigen Ansprüche, **dadurch gekennzeichnet, dass** sie Führungen (17) zum vertikalen Gleiten der Plattformen (13) umfasst.

6. Die Vorrichtung gemäß einem oder mehreren der obigen Ansprüche, **dadurch gekennzeichnet, dass** die Plattformen (13) auf der oberen Fußauflagefläche eine Messskala (28) zum Anzeigen des Grades der Spreizung der Füße und der Ferseneinheiten (29) zum Zwecke ihrer genauen Positionierung haben.

## Revendications

1. Appareil (10) pour évaluer la dysmétrie fonctionnelle de membres inférieurs, l'appareil (10) comportant :
- une structure de support (11) ayant deux plates-formes mobiles (13), qui sont mutuellement indépendantes et peuvent fléchir verticalement sous l'action d'une charge, au moins un bloc (14) constitué d'un matériau fléchissant élastiquement étant agencé sous chacune desdites plates-formes,
- des moyens complémentaires (31), qui coopèrent avec lesdites plates-formes (13), pour l'agencement vertical, par rapport à la ligne centrale de repose-pied, de la ligne lombo-sacrée du patient,
- des moyens (16) pour mesurer une dysmétrie,
- des moyens (30) pour mesurer les différences de charge subcalcanéenne,
- des moyens (26) pour bloquer et libérer lesdites plates-formes (13),
- des moyens (23) pour extraire les blocs (14) de ladite structure de support (11),
**caractérisé en ce que** lesdits moyens d'extraction (23) comportent un coulisseau (24) qui supporte lesdits blocs (14) et peut être extrait de ladite structure de support (11) en coulissant dans une direction qui est sensiblement parallèle au sol.

2. Appareil selon la revendication 1, **caractérisé en ce que** chacune des plates-formes (13) comporte une surface horizontale (18) avec une paroi latérale (19), sur la partie extérieure, et une surface d'appui (20), qui est superposée à la surface horizontale (18), pour le pied du patient, qui se situent au-dessus de deux blocs (14) qui sont agencés pratiquement entre les deux parois latérales (19) des deux plates-formes (13).

3. Appareil selon une ou plusieurs des revendications précédentes, **caractérisé en ce que** ladite surface horizontale (18) de chaque plate-forme (13) est pourvue d'une cellule dynamométrique (30), dans la région qui correspond à l'appui du talon, qui est adaptée pour détecter le poids du patient qui marche sur la plate-forme (13) respective.

4. Appareil selon une ou plusieurs des revendications précédentes, **caractérisé en ce que** lesdits moyens de mesure (16) comportent, pour chacune desdites plates-formes (13), un potentiomètre pour lire une dysmétrie.

5. Appareil selon une ou plusieurs des revendications précédentes, **caractérisé en ce qu'**il comporte des guides (17) pour le coulissement vertical desdites plates-formes (13).

6. Appareil selon une ou plusieurs des revendications précédentes, **caractérisé en ce que** lesdites plates-formes (13) ont, sur la surface de repose-pied supérieure, une échelle graduée (28) pour indiquer le degré de divarication des pieds et des unités de talon (29) pour leur positionnement précis.
